# EUROPÄISCHE PATENTSCHRIFT

(11) **EP 3 231 405 B1**
(45) Veröffentlichungstag und Bekanntmachung des Hinweises auf die Patenterteilung: **21.08.2019**
(21) Anmeldenummer: 16165643.4
(22) Anmeldetag: 15.04.2016
(51) Int. Cl.: A61F 13/42, G08B 31/00

(54) **VERFAHREN ZUR VORHERSAGE VON AUSSCHEIDUNGEN SOWIE VORRICHTUNG HIERZU**
METHOD FOR PREDICTING DEPOSIT FORMATION AND DEVICE FOR THE SAME
PROCEDE DE PREVISION D'EXCRETIONS ET DISPOSITIF ASSOCIE

(43) Veröffentlichungstag der Anmeldung: 18.10.2017
(73) Patentinhaber: Reifenhäuser GmbH & Co. KG Maschinenfabrik, 53844 Troisdorf (DE)
(72) Erfinder: Kunze, Bernd, 53773 Hennef (DE); Pomp, Philipp, 53359 Rheinbach (DE); Fett, Thomas, 53840 Troisdorf (DE); Rösner, Andreas, 53113 Bonn (DE)
(74) Vertreter: Rohmann, Michael

(56) Entgegenhaltungen:
- WO-A1-2008/023289
- WO-A1-2011/125003
- WO-A1-2013/061181
- WO-A2-2009/009803
- DE-T2- 69 930 853

## Beschreibung

Die Erfindung betrifft ein Verfahren zur Vorhersage von Ausscheidungen eines Windelträgers in einer Windel mit Hilfe eines Windelmoduls, wobei das Windelmodul einen Ausscheidungsfühler, einen Bewegungsfühler und eine Recheneinheit umfasst. Die Erfindung betrifft ferner ein Windelmodul zur Vorhersage von Ausscheidungen eines Windelträgers in einer Windel.

Windelmodule als solche sind aus DE 699 30 853 T2 bereits bekannt. Darin werden unter anderem Ausscheidungsfühler und Bewegungsfühler vorgeschlagen, um eine Frühwarn-Messvorrichtung zu schaffen. Solche vorhersagenden, auch proaktiv genannten Fühler können beispielsweise Haltung, Druck, Bewegung, Vibration, Kontraktion, Spannung, Durchblutung, Feuchtigkeit, Temperatur, Enzyme, Bakterien, pH-Werte, Leitfähigkeit, Widerstand, Kapazitanz, Induktanz und andere chemische, biochemische, biologische, mechanische oder elektrische Eigenschaften erfassen. Allerdings wird letztlich nur ein Bewegungsfühler empfohlen, welcher die Muskelkontraktionen des Analsphinkters erfasst. Demzufolge muss der Bewegungsfühler in unmittelbarer Nähe des Analsphinkters angeordnet sein, um dessen Aktivitäten und damit eine bevorstehende Ausscheidung zu erfassen. Dabei ist zu beachten, dass derartige Muskelkontraktionen ein sehr klares und deutliches Signal darstellen. Dies bedingt, dass mit einem entsprechend dicht an dem Analsphinkter angeordneten Bewegungsfühler das dahinter stehende Programm zur Erkennung der Kontraktionsmuster auf keine nennenswerten Probleme trifft. Das Programm muss nur in der Lage sein, eine im Mittel erhöhte Kontraktionsamplitude festzustellen. Hierzu bedarf es lediglich eines voreingestellten Schwellwertes der Kontraktionsamplitude, welcher oberhalb der üblichen Spannbreite der Basisaktivität des Analsphinkters liegt.

Allerdings kann das bekannte Windelmodul ausschließlich feste Ausscheidungen vorhersagen. Wünschenswert ist es aber, dass jegliche und insbesondere flüssige Ausscheidungen vorhergesagt werden können, so dass die Kinder meistens rechtzeitig von ihrer Windel befreit werden und zu dem Kindertopf gelangen. Dies würde den Lernvorgang erheblich beschleunigen, wodurch die Kinder teilweise um Jahre früher von der Windel entwöhnt würden. In der Folge könnte der Windelverbrauch drastisch verringert und die Umwelt entsprechend stärker geschont werden. Darüber hinaus ist auch die zielgenaue Anordnung des Bewegungsfühlers in unmittelbarer Nähe des Analsphinkters schwierig. Denn auf Grund des teilweise erheblichen Bewegungsdranges von kleinen Kindern können die Bewegungsfühler leicht verrutschen und so die Erfassung der Muskelkontraktionen verunmöglichen.

In der WO 2008/023289 A1 ist ein Windelmodul beschrieben, welches der Vorhersage von Ausscheidungen eines Windelträgers dient. Das Windelmodul umfasst ein Gehäuse mit einem Mikroprozessor sowie einen Ausscheidungsfühler. Optional kann das Windelmodul auch einen Bewegungsfühler in Form eines Beschleunigungssenors aufweisen. Das Windelmodul ist somit wenigstens in Teilen an der Windel angeordnet. Anhand typischer Ausscheidungszeiten sagt das Windelmodul die jeweils nächste bevorstehende Ausscheidung vorher. Dabei erhält das Windelmodul über den Ausscheidungsfühler eine automatisierte Rückkopplung, sodass die Vorhersage laufend angepasst werden kann. Im Falle einer vorhergesagten, bevorstehenden Ausscheidung wird zudem ein Vorhersagesignal ausgegeben. Nachteilig an diesem Windelmodul ist, dass die Vorhersagen nur auf der Ermittlung der typischen Ausscheidungszeiten beruhen, sodass die Vorhersagen verhältnismäßig statisch und damit unzuverlässig sind. Trinkt beispielsweise ein Kind zu einer Mahlzeit deutlich mehr als sonst, würde die dazugehörige Ausscheidung früher als sonst eintreten. Dies zu erkennen aber ist mit dem vorbekannten Windelmodul nicht möglich, weil es sich nur an den typischen Ausscheidungszeiten orientiert.

Aufgabe der vorliegenden Erfindung ist es daher, ein Verfahren zu schaffen, mit welchem - vorzugsweise wenigstens flüssige - Ausscheidungen, insbesondere von Kindern, zuverlässig vorhergesagt werden können. Ebenso liegt der Erfindung das technische Problem zugrunde, eine entsprechende Vorrichtung anzugeben.

Zur Lösung dieses technischen Problems lehrt die Erfindung ein Verfahren zur Vorhersage von Ausscheidungen eines Windelträgers in einer Windel mit Hilfe eines Windelmoduls, wobei das Windelmodul einen Ausscheidungsfühler, einen Bewegungsfühler und eine Recheneinheit umfasst, wobei das Windelmodul wenigstens in Teilen an der Windel angeordnet wird, wobei der Bewegungsfühler Bewegungsmuster des Windelträgers erfasst, wobei die Recheneinheit mit Hilfe des Ausscheidungsfühlers die Bewegungsmuster mit Daten des Ausscheidungsfühlers verknüpft, wobei im Falle von detektierten Ausscheidungen die Bewegungsmuster als maßgebliche Bewegungsmuster gespeichert und mit den, bevorzugt nachfolgend, detektierten Ausscheidungen verknüpft werden, wobei die Recheneinheit mit Hilfe der maßgeblichen Bewegungsmuster eine Bewegungsmustererkennung begründet und lernt, maßgebliche von nicht-maßgeblichen Bewegungsmustern zu unterscheiden, wobei die Bewegungsmuster mit Hilfe der Bewegungsmustererkennung derart klassifiziert werden, dass einige der Bewegungsmuster den maßgeblichen Bewegungsmustern zugeordnet werden, wodurch Vorhersagen zu bevorstehenden Ausscheidungen getroffen werden, wobei das Windelmodul im Falle einer Vorhersage ein Vorhersagesignal zur Warnung eines Benutzers aussendet, wobei einige der Vorhersignale von dem Benutzer überprüft und die Ergebnisse dieser Überprüfungen zur Verbesserung der Bewegungsmustererkennung von der Recheneinheit gespeichert werden.

Der Begriff "Ausscheidungsfühler" meint insbesondere Feuchtigkeitsfühler und/oder Temperaturfühler. Die Recheneinheit umfasst vorzugsweise einen Prozessor sowie weiter vorzugsweise einen Speicher. Der Ausdruck "in Teilen an der Windel angeordnet" meint, dass das Windelmodul auch zwei oder mehrere körperlich voneinander getrennte Teile aufweisen kann. Beispielsweise ist nur der Bewegungsfühler und vorzugsweise auch ein Teil des Ausscheidungsfühlers - bevorzugt in einem Gehäuse - an der Windel angeordnet, wohingegen sich die Recheneinheit nicht an der Windel, sondern in einem externen Gerät befinden kann. Es ist möglich, dass der Ausscheidungs- und der Bewegungsfühler über Funk mit der Recheneinheit kommunizieren. Gemäß einer anderen bevorzugten Ausführungsform befinden sich sowohl ein Teil des Ausscheidungsfühlers, der Bewegungsfühler als auch die Recheneinheit in einem Gehäuse, welches an der Windel angeordnet ist.

Der Begriff "Bewegungsmuster" meint insbesondere einen von dem Bewegungsfühler aufgenommenen und weiterverarbeiteten Datensatz. Der Datensatz umfasst bevorzugt den Verlauf einer Bewegungsamplitude über der Zeit. Der Ausdruck "Mustererkennung" umfasst insbesondere Merkmalsextraktion, Merkmalsreduktion und Klassifikation. Unter "Merkmalsextraktion" wird beispielsweise die Gewinnung von Merkmalen wie Frequenzen und Amplitudenverteilungen aus einem Bewegungsmuster verstanden. Unter "Merkmalsreduktion" wird insbesondere die Beschränkung der Gesamtheit der Merkmale auf wesentliche Merkmale verstanden, wobei die wesentlichen Merkmale bevorzugt besonders aussagekräftig sind. Unter "Klassifikation" wird insbesondere verstanden, ein zweites Bewegungsmuster an Hand der wesentlichen Merkmale einer bestimmten Klasse (beispielsweise keine Ausscheidung, flüssige Ausscheidung, feste Ausscheidung), zuzuordnen.

Unter dem Begriff "Vorhersagesignal" werden beispielsweise akustische und/oder optische und/oder haptische Signale verstanden. Dies kann beispielsweise ein heller Ton, ein Blinken einer LED oder ein Vibrieren sein. Bevorzugt wird das Vorhersagesignal mittels - beispielsweise - Funk oder Infrarot auf ein externes Gerät weitergeleitet, und dort in ein akustisches/optisches/haptisches Signal umgewandelt.

Der Erfindung liegt die Erkenntnis zugrunde, dass Säuglinge und Kleinkinder individuell typische Bewegungsmuster kurz vor den Ausscheidungen vollführen. Dabei unterscheiden sich die Bewegungsmuster der Kinder teilweise ganz erheblich. Einige Kinder neigen dazu, sich zu wälzen, während andere beispielsweise strampeln oder lediglich für eine bestimmte Zeit in einer Position verharren. Die Bandbreite der in Frage kommenden Bewegungsmuster hängt somit zunächst von dem einzelnen Kind als auch von der jeweiligen Ausscheidungsart ab. Es ist daher nicht möglich, allein mit Hilfe eines vordefinierten Satzes an Bewegungsmustern zuverlässige Vorhersagen treffen zu können. Es wurde aber gefunden, dass eine lernfähige Bewegungsmustererkennung sehr wohl in der Lage ist, zuverlässige Vorhersagen zu treffen. Die Lernfähigkeit beruht vor allem auf den Messungen des Ausscheidungsfühlers, über welchen mit der Bewegungsmustererkennung getroffene Vorhersagen überprüft werden können. Das Windelmodul verbessert sich hinsichtlich der Vorhersagen gewissermaßen von Windel zu Windel. So stellt sich bereits nach einigen Tagen ein spürbarer Lernerfolg ein, bis das Windelmodul nach einigen Wochen die Anlernphase beendet hat. Dann treffen Ausscheidungsvorhersagen regelmäßig mit großer Wahrscheinlichkeit zu. Ein weiterer großer Vorteil der Lernfähigkeit ist es, dass sich die Bewegungsmuster des Kindes im Laufe der Zeit noch verändern können. Doch solche Veränderungen werden innerhalb weniger Tage vom Windelmodul erfasst und in wieder zutreffende Vorhersagen umgesetzt. Bei konsequenter Anwendung sind die Kinder nicht selten bereits im ersten Lebensjahr von der Windel entwöhnt. Dies ist für den Verbraucher mit erheblichen wirtschaftlichen Vorteilen verbunden. Außerdem kann auf diese Weise eine sehr große Menge an Kunststoffmüll eingespart werden.

Vorzugsweise hat die Benutzerüberprüfung hinsichtlich der Verbesserung der Bewegungsmustererkennung Vorrang gegenüber den Daten des Ausscheidungsfühlers. Beispielsweise führt eine richtige bzw. falsche Vorhersage zu einer Hochstufung bzw. Herabstufung der Relevanz des der Vorhersage zu Grunde liegenden Bewegungsmusters.

Es ist möglich, dass das Windelmodul die Daten des Ausscheidungsfühlers hinsichtlich der Art der Ausscheidung unterscheidet. Zweckmäßigerweise umfasst das Windelmodul einen Feuchtigkeitsfühler und/oder einen Temperaturfühler. Es ist bevorzugt, dass die Art der Ausscheidung verknüpft wird mit ersten, zweiten, und vorzugsweise weiteren maßgeblichen Bewegungsmustern. Vorteilhafterweise werden die zweiten Bewegungsmuster den ersten, zweiten und vorzugsweise maßgeblichen Bewegungsmustern zugeordnet. Es ist vorteilhaft, wenn das Windelmodul der jeweiligen Art der Ausscheidung entsprechende Vorhersagesignale ausgibt.

Gemäß einer bevorzugten Ausführungsform sendet das Windelmodul im Falle einer detektierten Ausscheidung ein Ausscheidungssignal zum Hinweisen des Benutzers. Das Ausscheidungssignal bzw. die Ausscheidungssignale umfassen beispielsweise akustische und/oder optische und/oder haptische Signale. In Betracht kommen etwa ein heller Ton bzw. eine Tonfolge, ein Blinken einer LED oder ein Vibrieren. Bevorzugt wird das Ausscheidungssignal bzw. die Ausscheidungssignale mittels einer Kommunikationseinheit etwa per Funk oder Infrarot auf ein mobiles elektronisches Gerät übertragen, wo es in ein akustisches/haptisches Signal umgesetzt wird. Es ist zweckmäßig, dass sich das Ausscheidungssignal bzw. die Ausscheidungssignale von dem Vorhersagesignal bzw. von den Vorhersagesignalen unterscheidet. Zweckmäßigerweise unterscheiden sich die Ausscheidungssignale voneinander, so dass für den Benutzer die Art der Ausscheidung kenntlich gemacht wird.

Es ist bevorzugt, dass auf einige der Ausscheidungssignale hin der Benutzer überprüft, ob und vorzugsweise welche Ausscheidung vorliegt, wobei die Ergebnisse dieser Überprüfungen zwecks Verbesserung einer Ausscheidungsmustererkennung zur Detektierung von Ausscheidungen von der Recheneinheit gespeichert werden. Dem liegt die Erkenntnis zu Grunde, dass eine lernfähige Ausscheidungsmustererkennung eine Ausscheidung bzw. die Art von Ausscheidungen zuverlässiger erfasst.

Es liegt im Rahmen der Erfindung, dass das Windelmodul einen vorgegebenen Satz an Bewegungsmustern und/oder Ausscheidungsmustern enthält. Der Begriff "vorgegeben" meint insbesondere, dass die Bewegungs- bzw. Ausscheidungsmuster ab Werk bzw. vor Verkauf an Endkunden bzw. als nachträgliches *Update* in dem Windelmodul gespeichert werden. Dies kann die Lernfähigkeit beschleunigen, da so von Anfang an für die Bewegungsmustererkennung bzw. Ausscheidungsmustererkennung erforderlichen Bewegungsmuster bzw. Ausscheidungsmuster vorliegen.

Es ist vorteilhaft, wenn das Windelmodul einen vorgegebenen Satz an Filtern zur Verbesserung der Unterscheidung zwischen maßgeblichen und nicht-maßgeblichen Bewegungsmustern und/oder Ausscheidungsmustern aufweist. Insbesondere werden herausgefilterte Bewegungsmuster nicht zum Anlernen der Bewegungsmustererkennung herangezogen. Die Filter können beispielsweise extreme Bewegungsmuster heraussortieren. Es ist bevorzugt, wenn die Filter beispielsweise Schwellwerte von Bewegungsamplituden festlegen, wobei Bewegungsamplituden oberhalb dieser Schwellwerte dazu führen, dass die dazugehörigen Bewegungsmuster nicht zum Lernen der Bewegungsmustererkennung herangezogen werden. Es ist zweckmäßig, dass die Filter Wertober- und/oder Wertuntergrenzen von Bewegungsamplituden definieren. Auf diese Weise werden extreme Bewegungen von vorneherein aussortiert, so dass beispielsweise Sprünge des Kindes oder Drehungen auf der Wickelkommode gar nicht erst zum Lernen herangezogen werden.

Gemäß einer ganz besonders bevorzugten Ausführungsform folgen die mit den Bewegungsmustern verknüpften Daten des Ausscheidungsfühlers den Bewegungsmustern jeweils zeitlich nach. Zweckmäßigerweise liegen die nachfolgenden Daten des Ausscheidungsfühlers innerhalb einer vorgegebenen Zeitspanne hinter den Bewegungsmustern. Die Zeitspanne beträgt beispielsweise eine Minute.

Gemäß einer sehr bevorzugten Ausführungsform umfasst das Windelmodul eine Kommunikationseinheit zur Kommunikation mit mobilen elektronischen Geräten. Die Kommunikationseinheit ist beispielsweise in der Lage, Funksignale zu senden und zu empfangen. In Betracht kommen etwa WLAN, Bluetooth oder GSM-Funksignale. Es ist aber auch möglich, dass die Kommunikationseinheit Infrarotstrahlung sendet und empfängt. Unter "Kommunikationseinheit" wird insbesondere eine Antenne samt zugehöriger Elektronik verstanden. Es liegt im Rahmen der Erfindung, dass das Windelmodul ein Zeitglied aufweist. Das Zeitglied befindet sich vorteilhafterweise in der Recheneinheit und weiter vorzugsweise in der Peripherie der Recheneinheit.

Vorteilhafterweise ist der Ausscheidungsfühler ein Feuchtigkeitsfühler. Bevorzugt umfasst der Ausscheidungsfühler ein flächiges Element, wobei vorzugsweise eine erste Kantenseite des flächigen Elementes länger ist als eine zweite Kantenseite. Zweckmäßigerweise sind elektrische Leiter an dem flächigen Element angeordnet, wobei die elektrischen Leiter vorzugsweise eine Widerstand oder eine Kapazität zwischen den elektrischen Leitern erfassen. Bevorzugterweise erstreckt sich wenigstens einer der elektrischen Leiter über wenigstens die Hälfte der Länge des flächigen Elementes.

Es ist vorteilhaft, dass ein Teil des Ausscheidungsfühlers reversibel und/oder irreversibel an dem Windelmodul angebracht wird. Vorzugsweise wird ein Teil des Ausscheidungsfühlers, insbesondere das flächige Element, mittels Drucckontaktierung, beispielsweise durch eine Federklemme, an einem Gehäuse des Windelmoduls befestigt. Zweckmäßigerweise ist der Ausscheidungsfühler so ausgebildet, dass er hygienisch zu reinigen ist.

Gemäß einer besonders bevorzugten Ausführungsform wird das Windelmodul reversibel und/oder irreversibel, beispielsweise mit einem Klettelement und insbesondere mit einem Klettband, an der Windel befestigt. Gemäß einer anderen Ausführungsform wird das Windelmodul mittels einer Klemme zum Einhängen des Windelmoduls an einem Rand der Windel befestigt.

Es ist vorteilhaft, dass das Windelmodul eine Schnittstelle zur Bedienung aufweist. Gemäß einer ersten Ausführungsform ist die Schnittstelle zur Bedienung an dem Gehäuse angeordnet und umfasst vorzugsweise wenigstens ein Eingabe- und/oder Ausgabeelement wie beispielsweise einen Bildschirm und/oder Leuchtdioden und/oder Knöpfe. Gemäß einer anderen Ausführungsform ist die Schnittstelle zur Bedienung auf einem mobilen elektronischen Gerät, angeordnet und kann folglich sämtliche Bedienelemente des mobilen elektronischen Gerätes, benutzen. Vorzugsweise umfasst das Windelmodul eine Schnittstelle zur Bedienung an dem Gehäuse und eine Schnittstelle zur Bedienung an dem mobilen elektronischen Gerät.

Gemäß einer bevorzugten Ausführungsform erfasst der Bewegungsfühler Drehbewegungen und/oder translatorische Bewegungen. Gemäß einer besonders bevorzugten Ausführungsform erfasst der Bewegungsfühler Drehbewegungen und translatorische Bewegungen. Der Bewegungsfühler kann einen Beschleunigungs- und/oder Magnetfeldsensor und/oder ein Gyroskop aufweisen. Der Bewegungsfühler ist gemäß einer bevorzugten Ausführungsform ein Inertialsensor.

Es ist möglich, dass die Recheneinheit in den an der Windel angeordneten Teilen und/oder in den nicht an der Windel angeordneten Teilen des Windelmoduls enthalten ist. Die Recheneinheit kann lediglich an der Windel, lediglich an einem mobilen elektronischen Gerät oder an beiden - in wie auch geteilter Form - Elementen gleichzeitig angeordnet sein.

Zur Lösung des technischen Problems lehrt die Erfindung ein Windelmodul zur Vorhersage von Ausscheidungen eines Windelträgers in einer Windel, insbesondere ein Windelmodul gemäß dem erfindungsgemäßen Verfahren, wobei das Windelmodul einen Ausscheidungsfühler, einen Bewegungsfühler und eine Recheneinheit umfasst, wobei das Windelmodul wenigstens in Teilen an der Windel angeordnet wird, wobei die Recheneinheit so ausgebildet ist, dass sie mit Hilfe des Ausscheidungsfühlers vom Bewegungsfühler erfasste Bewegungsmuster mit Daten des Ausscheidungsfühlers verknüpfen kann, wobei im Falle von detektierten Ausscheidungen die Bewegungsmuster als maßgebliche Bewegungsmuster gespeichert und mit den detektierten Ausscheidungen verknüpft werden, wobei die Recheneinheit so ausgebildet ist, dass sie mit Hilfe der maßgeblichen Bewegungsmuster eine Bewegungsmustererkennung begründen kann und lernen kann, maßgebliche von nicht-maßgeblichen Bewegungsmustern zu unterscheiden, wobei die Bewegungsmuster mit Hilfe der Bewegungsmustererkennung derart klassifiziert werden, dass einige der Bewegungsmuster den maßgeblichen Bewegungsmustern zugeordnet werden, wodurch Vorhersagen zu bevorstehenden Ausscheidungen getroffen werden können, wobei das Windelmodul im Falle einer der Vorhersagen ein Vorhersagesignal zur Warnung eines Benutzers aussenden kann,

wobei einige der Vorhersignale von dem Benutzer überprüft und die Ergebnisse dieser Überprüfungen zur Verbesserung der Bewegungsmustererkennung von der Recheneinheit gespeichert werden.

Nachfolgend wird die Erfindung anhand einer lediglich ein Ausführungsbeispiel darstellenden Zeichnung detailliert erläutert. Es zeigen in schematischer Darstellung
- Fig. 1: ein erfindungsgemäßes, an eine Windel angebrachtes Windelmodul in perspektivischer Darstellung,
- Fig. 2: das Windelmodul aus Fig. 1 in perspektivischer Darstellung mit Blick auf die Vorderseite,
- Fig. 3: das Windelmodul aus den Fig. 1 und 2 in perspektivischer Darstellung mit Blick auf die Rückseite und
- Fig. 4: ein Blockdiagramm des Windelmoduls aus den Fig. 1 bis 3.

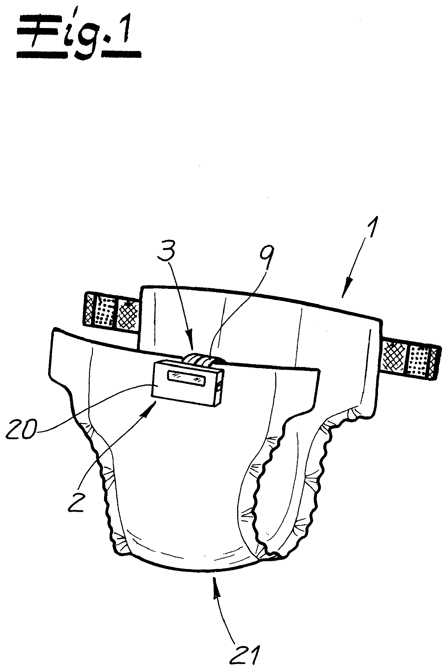

In Fig. 1 ist eine Windel 1 dargestellt, an welche Windel 1 ein erfindungsgemäßes Windelmodul 2 mittels eines hier nicht dargestellten Klettverschlusses angebracht ist. Das Windelmodul 2 umfasst einen Ausscheidungsfühler 3, welcher vor allem durch ein saugfähiges, länglich ausgebildetes und flächiges Element 9 gebildet wird. Das flächige Element 9 erstreckt sich in diesem Ausführungsbeispiel von einem Gehäuse 20 des Windelmoduls 2 über den oberen Rand der Windel 1 und verläuft von dort entlang der Innenseite der Windel 1 bis zu einem Schrittbereich 21 der Windel 1. Hierzu weist das flächige Element 9 auf einer Seite ein nicht dargestelltes Klettelement auf.

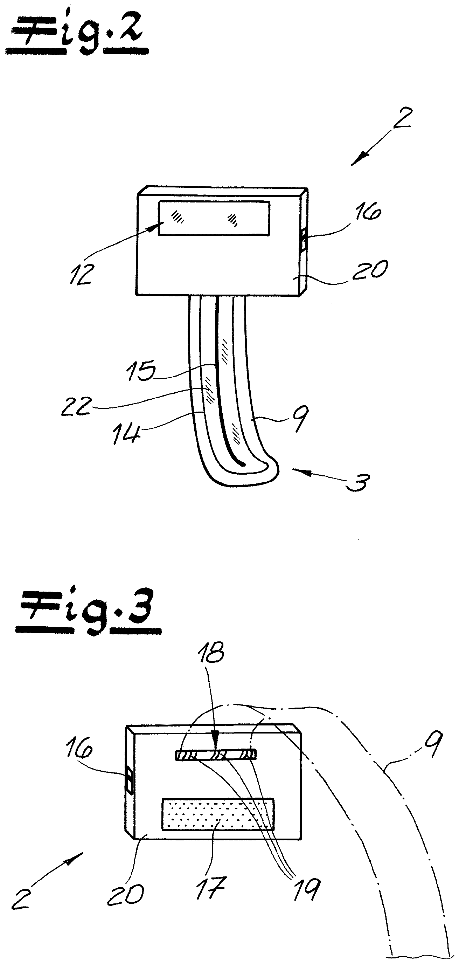

In Fig. 2 ist das Windelmodul 2 ohne die Windel 1 abgebildet. Das Gehäuse 20 weist einen Ein-/aus-Schalter 16 sowie eine Schnittstelle 12 zur Bedienung des Windelmoduls 2 auf. Das flächige Element 9 ist weist zwei elektrische Leiter 14, 15 auf, wobei der Leiter 14 den Leiter 15 umschließt. In diesem Ausführungsbeispiel ist der Ausscheidungsfühler 3 ein Feuchtigkeitsfühler, welcher über Widerstandsmessungen eines feuchtigkeitsabhängigen Widerstandes 22 zwischen den elektrischen Leitern 14, 15 in der Lage ist, die Feuchtigkeit in der Windel 1 zu messen. Aufgrund einer Feuchtigkeitszunahme in der Umgebung des Widerstandes 22 wird der elektrische Widerstandswert zwischen den elektrischen Leitern 14, 15 verringert. Folglich kann bei Anlegen einer Spannung zwischen den elektrischen Leitern 14, 15 im Falle eines Absinkens des gemessenen Widerstandswertes von einem Anstieg der Feuchtigkeit in der Windel 1 ausgegangen werden.

In Fig. 3 ist lediglich das Gehäuse 20 des Windelmoduls 2 mit Blick auf dessen Rückseite dargestellt. Zu erkennen ist ein Klettband 17, welches für einen stabilen Halt der Windel 1 sorgt. Außerdem ist eine Aussparung 18 ersichtlich, in welche ein Ende des flächigen Elementes 9 hineingeschoben werden kann. Hierbei werden die elektrischen Leiter 14, 15 des flächigen Elementes 9 mittels einer Druckkontaktierung 19 und vorzugsweise über Federklemmen mit dem Gehäuse 20 verbunden.

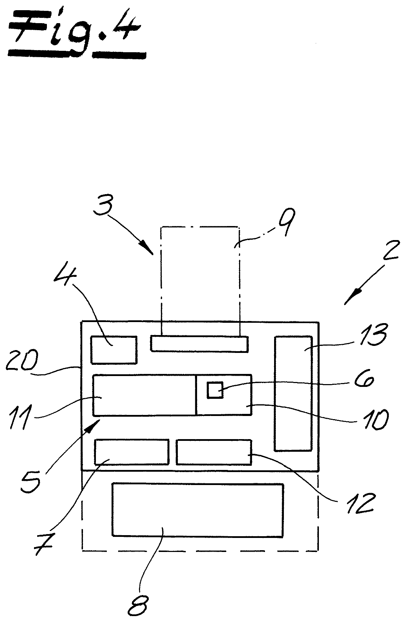

In Fig. 4 ist das Windelmodul 2 in einem Blockdiagramm veranschaulicht. Neben dem Ausscheidungsfühler 3 und der Schnittstelle 12 zur Bedienung enthält das Gehäuse 20 außerdem noch einen Bewegungsfühler 4, eine Recheneinheit 5, eine Kommunikationseinheit 7 sowie eine Spannungsversorgung 13. Der Bewegungsfühler 4 ist ein Inertialsensor, welcher in der Lage ist, sowohl Dreh- als auch translatorische Bewegungen zu erfassen. Hierdurch können die vor Ausscheidungen nicht unüblichen Wälz- als auch Strampelbewegungen erfasst werden. Die Recheneinheit 5 weist einen Prozessor 10, einem Speicher 11 sowie ein Zeitglied 6 auf, wobei der Prozessor 10 außerdem noch einen Zeitgeber 6 enthält.

Die Kommunikationseinheit 7 ist in diesem Ausführungsbeispiel eine WLAN-Schnittstelle und kann folglich ohne weiteres mit mobilen elektronischen Geräten 8 wie beispielsweise Smartphones oder Tablets verbunden werden. In diesem Ausführungsbeispiel umfasst das Windelmodul 2 auch ein Programm auf einem mobilen elektronischen Gerät 8 in Form eines Smartphones sowie das mobile elektronische Gerät 8 selber. Das Programm kommuniziert über eine Kommunikationseinheit des Smartphones und die Kommunikationseinheit 7 im Gehäuse 20 mit der Recheneinheit 5. In diesem Ausführungsbeispiel ist das mobile elektronische Gerät 8 folglich Bestandteil des Windelmoduls 2, wird aber nicht von dem Gehäuse 20 umschlossen. Dieser Umstand ist mit der gestrichelten Linie angedeutet.

Bei dem Anlegen der Windel 1 an den Windelträger wird zugleich auch das Gehäuse 20 mitsamt einem neuen, eingesetzten flächigen Element 9 mittels des Klettbandes 17 an der Windel 1 befestigt. Das flächige Element 9 wird um den oberen Rand der Windel 1 gebogen und mittels seines Klettelementes an der Innenwand der Windel 1 bis hinunter zum Schrittbereich 21 befestigt. Mit Verschließen der Windel 1 und Betätigen des Ein-/Ausschalters 16 ist das Windelmodul 2 nun betriebsbereit.

Das Windelmodul 2 nimmt mit Hilfe des Bewegungsfühlers 3 und des Zeitgebers 6 fortlaufend Bewegungsmuster des Windelträgers auf. Sobald der Ausscheidungsfühler 3 ein schnelles Absinken des Widerstandswertes des elektrischen Widerstandes 22 bemerkt, wird die solcherart detektierte Ausscheidung mit dem vorangegangenen maßgeblichen Bewegungsmuster verknüpft. Im Laufe der Zeit werden zahlreiche Verknüpfungen dieser Art vorgenommen.

Über die Geschwindigkeit des Absinkens des Widerstandswertes kann die Recheneinheit 5 abschätzen, ob es sich hier um Schweiß, eine feste oder eine flüssige Ausscheidung handelt. Dabei werden insbesondere die den festen bzw. flüssigen Ausscheidungen vorangegangen Bewegungsmuster als maßgebliche Bewegungsmuster gespeichert. Die maßgeblichen Bewegungsmuster dienen als Grundlage für eine Bewegungsmustererkennung. Das Programm der Bewegungsmustererkennung befindet sich im Speicher 11 der Recheneinheit 5.

Aus der Vielzahl der gespeicherten Bewegungsmuster werden in einer Merkmalsextraktion zunächst aus Bewegungsamplitude-Zeit-Verläufen verschiedene Merkmale wie beispielsweise Frequenzen und Amplitudenverteilungen der jeweiligen Bewegungsmuster ermittelt. Sodann werden in einer Merkmalsreduktion die aussagekräftigsten Merkmale herausgesucht, welche besonders stark mit dem jeweiligen Ausscheidungstyp korrelieren.

Nach einer kurzen Anlernzeit hat die Bewegungsmustererkennung ausreichend viele Bewegungsmuster gesammelt, um eine Kombination der aussagekräftigsten Merkmale zusammenzustellen. Nun können die Bewegungsmuster anhand der aussagekräftigen Merkmale hinsichtlich der Art der Ausscheidung klassifiziert werden. Wird beispielsweise ein Bewegungsmuster erfasst, welches typische Merkmale einer bevorstehenden flüssigen Ausscheidung enthält, so wird die Recheneinheit 5 über die Kommunikationseinheit 7 ein Signal an das mobile elektronische Gerät 8 senden, wodurch der Benutzer vor einer bevorstehenden flüssigen Ausscheidung gewarnt wird. Der Benutzer kann nun den Windelträger, beispielsweise ein Kind, auf einen Kindertopf setzen. Mit der Zeit wird das Kind hierdurch sehr schnell von der Windel 1 entwöhnt, der Windelverbrauch drastisch gesenkt und die Umwelt entsprechend geschont.

Erfindungsgemäß kann der Benutzer - speziell in der Anlernphase - nach einem Vorhersagesignal überprüfen, ob und vorzugsweise wenn ja, welche Ausscheidung vorliegt. Sodann kann der Benutzer das Ergebnis dieser Überprüfung mittels seines mobilen elektronischen Gerätes 8 eingeben, wodurch die von dem Windelmodul 2 getroffenen Vorhersagen entweder bestätigt oder aber verneint werden. Im Falle der Bestätigung wird ein Bewegungsmuster bzw. ein bestimmtes Merkmal des Bewegungsmusters entsprechend hochgestuft. Auf diese Weise wird die Lernfähigkeit der Bewegungsmustererkennung noch erhöht.

Außerdem versendet das Windelmodul 2 vorteilhafterweise im Falle einer detektierten Ausscheidung in der Windel 1 ein Ausscheidungssignal. Dieses Ausscheidungssignal unterscheidet sich von dem Vorhersagesignal, so dass der Benutzer sogleich informiert ist. Im Übrigen kann der Benutzer auch die Richtigkeit der detektierten Ausscheidungsart bestätigen oder verneinen, so dass das Windelmodul 2 auch hinsichtlich der Ausscheidungsmustererkennung lernfähig ist.

## Patentansprüche

1. Verfahren zur Vorhersage von Ausscheidungen eines Windelträgers in einer Windel (1) mit Hilfe eines Windelmoduls (2), wobei das Windelmodul (2) einen Ausscheidungsfühler (3), einen Bewegungsfühler (4) und eine Recheneinheit (5) umfasst, wobei das Windelmodul (2) wenigstens in Teilen an der Windel (1) angeordnet wird, wobei der Bewegungsfühler (4) Bewegungsmuster des Windelträgers erfasst,
wobei die Recheneinheit (5) mit Hilfe des Ausscheidungsfühlers (3) die Bewegungsmuster mit Daten des Ausscheidungsfühlers (3) verknüpft, wobei im Falle von detektierten Ausscheidungen die Bewegungsmuster als maßgebliche Bewegungsmuster gespeichert und mit den, bevorzugt nachfolgend, detektierten Ausscheidungen verknüpft werden,
wobei die Recheneinheit (5) mit Hilfe der maßgeblichen Bewegungsmuster eine Bewegungsmustererkennung begründet, und lernt, maßgebliche von nicht-maßgeblichen Bewegungsmustern zu unterscheiden,
wobei die Bewegungsmuster mit Hilfe der Bewegungsmustererkennung derart klassifiziert werden, dass einige der Bewegungsmuster den maßgeblichen Bewegungsmustern zugeordnet werden, wodurch Vorhersagen zu bevorstehenden Ausscheidungen getroffen werden,
wobei das Windelmodul (2) im Falle einer Vorhersage ein Vorhersagesignal zur Warnung eines Benutzers aussendet,
**dadurch gekennzeichnet, dass**
einige der Vorhersagesignale von dem Benutzer überprüft werden und die Ergebnisse dieser Überprüfung zur Verbesserung der Bewegungsmustererkennung von der Recheneinheit (5) gespeichert werden.

2. Verfahren nach Anspruch 1, wobei das Windelmodul (2) im Falle einer detektierten Ausscheidung ein Ausscheidungssignal zum Hinweisen des Benutzers sendet.

3. Verfahren nach Anspruch 2, wobei der Benutzer auf einige der Ausscheidungssignale hin überprüft, ob eine Ausscheidung vorliegt und wobei die Ergebnisse dieser Überprüfungen zwecks Verbesserung einer Ausscheidungsmustererkennung zu Detektierung von Ausscheidungen von der Recheneinheit (5) gespeichert werden.

4. Verfahren nach einem der Ansprüche 1 bis 3, wobei das Windelmodul (2) einen vorgegebenen Satz an Bewegungsmustern und/oder Ausscheidungsmustern enthält.

5. Verfahren nach einem der Ansprüche 1 bis 4, wobei das Windelmodul (2) einen vorgegeben Satz an Filtern zur Verbesserung der Unterscheidung zwischen maßgeblichen und nicht-maßgeblichen Bewegungsmustern und/oder Ausscheidungsmustern aufweist.

6. Verfahren nach einem der Ansprüche 1 bis 5, wobei die mit den Bewegungsmustern verknüpften Daten des Ausscheidungsfühlers (3) den Bewegungsmustern zeitlich nachfolgen.

7. Verfahren nach einem der Ansprüche 1 bis 6, wobei das Windelmodul (2) eine Kommunikationseinheit (7) zur Kommunikation mit mobilen elektronischen Geräten (8) umfasst.

8. Verfahren nach einem der Ansprüche 1 bis 7, wobei der Ausscheidungsfühler (3) einen Feuchtigkeitsfühler aufweist.

9. Verfahren nach einem der Ansprüche 1 bis 8, wobei ein Teil des Ausscheidungsfühlers (3) reversibel oder irreversibel an dem Windelmodul (2) befestigt wird.

10. Verfahren nach einem der Ansprüche 1 bis 9, wobei das Windelmodul (2) reversibel oder irreversibel an der Windel (1) angebracht wird.

11. Verfahren nach einem der Ansprüche 1 bis 10, wobei das Windelmodul (2) eine Schnittstelle (12) zur Bedienung aufweist.

12. Verfahren nach einem der Ansprüche 1 bis 11, wobei der Bewegungsfühler (4) Drehbewegungen und/oder translatorische Bewegungen erfasst.

13. Verfahren nach einem Ansprüche 1 bis 12, wobei die Recheneinheit (5) in den an der Windel (1) angeordneten Teilen und/oder in den nicht an der Windel (1) angeordneten Teilen des Windelmoduls (2) enthalten ist.

14. Windelmodul (2) zur Vorhersage von Ausscheidungen eines Windelträgers in einer Windel (1), insbesondere gemäß einem Ansprüche 1 bis 13, wobei das Windelmodul (2) einen Ausscheidungsfühler (3), einen Bewegungsfühler (4) und eine Recheneinheit (5) umfasst wobei das Windelmodul (2) wenigstens in Teilen an der Windel (1) angeordnet wird, wobei die Recheneinheit (5) so ausgebildet ist, dass sie mit Hilfe des Ausscheidungsfühlers (3) vom Bewegungsfühler (4) erfasste Bewegungsmuster mit Daten des Ausscheidungsfühlers (3) verknüpfen kann, wobei im Falle von detektierten Ausscheidungen die Bewegungsmuster als maßgebliche Bewegungsmuster gespeichert und mit den detektierten Ausscheidungen verknüpft werden,
wobei die Recheneinheit (5) so ausgebildet ist, dass sie mit Hilfe der maßgeblichen Bewegungsmuster eine Bewegungsmustererkennung begründen kann und lernen kann, maßgebliche von nicht-maßgeblichen Bewegungsmustern zu unterscheiden,
wobei die Bewegungsmuster mit Hilfe der Bewegungsmustererkennung derart klassifiziert werden, dass einige der Bewegungsmuster den maßgeblichen Bewegungsmustern zugeordnet werden, wodurch Vorhersagen zu bevorstehenden Ausscheidungen getroffen werden können, wobei das Windelmodul (2) im Falle einer der Vorhersagen ein Vorhersagesignal zur Warnung eines Benutzers aussenden kann,
**dadurch gekennzeichnet, dass**
einige der Vorhersagesignale von dem Benutzer überprüft werden und die Ergebnisse dieser Prüfung zur Verbesserung der Bewegungsmustererkennung von der Recheneinheit (5) gespeichert werden können.

## Claims

1. A method for predicting excretions of a nappy wearer in a nappy (1) with the aid of a nappy module (2),
wherein the nappy module (2) comprises an excretion sensor (3), a motion sensor (4) and a processing unit (5), wherein the nappy module (2) is arranged at least in parts on the nappy (1), wherein the motion sensor (4) detects movement patterns of the nappy wearer,
wherein the processing unit (5) with the aid of the excretion sensor (3) links the movement patterns to data of the excretion sensor (3), wherein in the case of detected excretions, the movement patterns are stored as definitive movement patterns and are linked to the preferably subsequently detected excretions,
wherein the processing unit (5) substantiates with the aid of the definitive movement patterns a movement pattern recognition and learns to distinguish definitive from non-definitive movement patterns,
wherein the movement patterns are classified with the aid of the movement pattern recognition in such a manner that some of the movement patterns are assigned to the definitive movement patterns, with the result that predictions on imminent excretions are made, wherein in the case of a prediction, the nappy module (2) outputs a prediction signal to warn a user,
**characterized in that** some of the prediction signals are checked by the user and the results of these checks are stored to improve the movement pattern recognition by a processing unit (5).

2. The method according to claim 1, wherein in the case of a detected excretion, the nappy module (2) transmits an excretion signal to inform the user.

3. The method according to claim 2, wherein after some of the excretion signals the user checks whether an excretion is present and wherein the results of these checks are stored for the purpose of improving an excretion pattern recognition for detecting excretions by the processing unit (5).

4. The method according to one of claims 1 to 3, wherein the nappy module (2) contains a predefined set of movement patterns and/or excretion patterns.

5. The method according to one of claims 1 to 4, wherein the nappy module (2) has a predefined set of filters for improving the differentiation between definitive and non-definitive movement patterns and/or excretion patterns.

6. The method according to one of claims 1 to 5, wherein the data of the excretion sensor (3) linked to the movement patterns follow the movement patterns in time.

7. The method according to one of claims 1 to 6, wherein the nappy module (2) comprises a communication unit (7) for communication with mobile electronic devices (8) .

8. The method according to one of claims 1 to 7, wherein the excretion sensor (3) comprises a moisture sensor.

9. The method according to one of claims 1 to 8, wherein a part of the excretion sensor (3) is fastened reversibly or irreversibly on the nappy module (2).

10. The method according to one of claims 1 to 9, wherein the nappy module (2) is mounted reversibly or irreversibly on the nappy (1).

11. The method according to one of claims 1 to 10, wherein the nappy module (2) has an interface (12) for operation.

12. The method according to one of claims 1 to 11, wherein the motion sensor (4) detects twisting movements and/or translational movements.

13. The method according to one of claims 1 to 12, wherein the processing unit (5) is contained in the parts arranged on the nappy (1) and/or in the parts of the nappy module (2) not arranged on the nappy (1).

14. A nappy module (2) for predicting excretions of a nappy wearer in a nappy (1), in particular according to one of claims 1 to 13, wherein the nappy module (2) comprises an excretion detector (3), a motion sensor (4) and a processing unit (5), wherein the nappy module (2) is arranged at least in parts on the nappy (1), wherein the processing unit (5) is configured such that, with the aid of the excretion detector (3), it can link movement patterns detected by the motion sensor (4) to data of the excretion detector (3), in which case the movement patterns are stored as definitive movement patterns when excretions are detected and associated with the excretions detected,
wherein the processing unit (5) is configured such that it can substantiate a movement-pattern recognition with the aid of the definitive movement patterns and can learn to distinguish definitive from non-definitive movement patterns,
wherein the movement patterns are classified with the aid of movement-pattern recognition in such a manner that several of the movement patterns are associated with the definitive movement patterns, with the result that predictions can be made regarding imminent excretions, wherein the nappy module (2) can transmit a prediction signal to warn a user in the event of one of the predictions,
**characterized in that**
some of the prediction signals are checked by the user and the results of this check can be stored to improve the movement pattern recognition by the processing unit (5).

## Revendications

1. Procédé destiné à prévoir des excrétions d'un porteur de couche dans une couche (1) à l'aide d'un module de couche (2), le module de couche (2) comprenant une sonde d'excrétions (3), un capteur de mouvements (4) et une unité de calcul (5), lors duquel on place le module de couche (2) au moins par parties sur la couche (1), le capteur de mouvements (4) détectant des modèles de mouvements du porteur de couche,
à l'aide de la sonde d'excrétions (3), l'unité de calcul (5) associant les modèles de mouvements avec des données de la sonde d'excrétions (3), dans le cas où des excrétions sont détectées, les modèles de mouvements étant mémorisés en tant que modèles de mouvements déterminants et étant associés avec des excrétions détectées, de préférence par la suite,
l'unité de calcul (5) fondant à l'aide des modèles de mouvements déterminants une identification des modèles de mouvements et apprenant à différencier des modèles de mouvements déterminants de modèles de mouvements non déterminants,
les modèles de mouvements étant classifiés à l'aide de l'identification des modèles de mouvements, de telle sorte que certains des modèles de mouvements soient affectés aux modèles de mouvements déterminants, suite à quoi des prévisions sont faites pour des futures excrétions,
dans le cas d'une prévision, le module de couche (2) émettant un signal prévisionnel destiné à avertir un utilisateur,
**caractérisé en ce que** certains des signaux prévisionnels sont vérifiés par l'utilisateur et les résultats de ladite vérification sont mémorisés pour améliorer l'identification des modèles de mouvements par l'unité de calcul (5).

2. Procédé selon la revendication 1, dans le cas de détection d'une excrétion, le module de couche (2) envoyant un signal d'excrétion pour informer l'utilisateur.

3. Procédé selon la revendication 2, après certains signaux d'excrétion, l'utilisateur vérifiant la présence d'une excrétion et les résultats desdites vérifications étant mémorisés par l'unité de calcul (5) aux fins d'amélioration d'une identification de modèle d'excrétion pour la détection d'excrétions.

4. Procédé selon l'une quelconque des revendications 1 à 3, le module de couche (2) contenant un jeu prédéfini de modèles de mouvements et/ou de modèles d'excrétions.

5. Procédé selon l'une quelconque des revendications 1 à 4, le module de couche (2) comportant un jeu prédéfini de filtres destinés à améliorer la différentiation entre des modèles de mouvements et/ou modèles d'excrétions déterminants et non déterminants.

6. Procédé selon l'une quelconque des revendications 1 à 5, en les données de la sonde d'excrétions (3) associés aux modèles de mouvements étant consécutifs dans le temps aux modèles de mouvements.

7. Procédé selon l'une quelconque des revendications 1 à 6, le module de couche (2) comprenant une unité de communication (7), destinée à communiquer avec des appareils (8) électroniques mobiles.

8. Procédé selon l'une quelconque des revendications 1 à 7, la sonde d'excrétions (3) comportant une sonde d'humidité.

9. Procédé selon l'une quelconque des revendications 1 à 8, une partie de la sonde d'excrétions (3) étant fixée de manière réversible ou irréversible sur le module de couche (2).

10. Procédé selon l'une quelconque des revendications 1 à 9, le module de couche (2) étant monté de manière réversible ou irréversible sur la couche (1).

11. Procédé selon l'une quelconque des revendications 1 à 10, le module de couche (2) comportant une interface (12) de commande.

12. Procédé selon l'une quelconque des revendications 1 à 11, le capteur de mouvements (4) détectant des mouvements de rotation et/ou des mouvements en translation.

13. Procédé selon l'une quelconque des revendications 1 à 12, l'unité de calcul (5) étant contenue dans les parties placées dans la couche (1) et/ou dans les parties non placées dans la couche (1) du module de couche (2).

14. Module de couche (2), destiné à prévoir des excrétions d'un porteur de couche dans une couche (1), notamment selon l'une quelconque des revendications 1 à 13, le module de couche (2) comprenant une sonde d'excrétions (3), un capteur de mouvements (4) et une unité de calcul (5), le module de couche (2) étant placé au moins par parties sur la couche (1), l'unité de calcul (5) étant conçue de sorte qu'à l'aide de la sonde d'excrétions (3), elle soit susceptible d'associer des modèles de mouvements détectés par le capteur de mouvements (4) avec des données de la sonde d'excrétion (3), dans le cas d'une détection d'excrétions, les modèles de mouvements étant mémorisés en tant que modèles de mouvements déterminants et étant associés à des excrétions détectées,
l'unité de calcul (5) étant conçue de telle sorte, qu'à l'aide des modèles de mouvements déterminants, elle soit susceptible de fonder une identification des modèles de mouvements et d'apprendre à différencier des modèles de mouvements déterminants des modèles de mouvement non déterminants,
les modèles de mouvements étant classifiés à l'aide de l'identification des modèles de mouvements, de telle sorte que certains des modèles de mouvements soient affectés aux modèles de mouvements déterminants, suite à quoi, des prévisions peuvent être faites pour des futures excrétions, dans le cas de l'une des prévisions, le module de couche (2) étant susceptible d'émettre un signal prévisionnel destiné à avertir un utilisateur,
**caractérisé en ce que** certains des signaux prévisionnels sont vérifiés par l'utilisateur et les résultats de ladite vérification peuvent être mémorisés pour l'amélioration de l'identification des modèles de mouvements par l'unité de calcul (5).
